# EUROPEAN PATENT APPLICATION

(11) **EP 2 489 724 A1**
(43) Date of publication of application: **22.08.2012**
(21) Application number: 10823486.5
(22) Date of filing: 15.10.2010
(51) Int. Cl.: C12N 1/19, C07K 16/18, C12N 15/09

(54) **HANSENULA POLYMORPHA CAPABLE OF PRODUCING ANTIBODY, PROCESS FOR PRODUCTION OF ANTIBODY UTILIZING SAME, AND ANTIBODY PRODUCED FROM SAME**

(30) Priority: 16.10.2009 JP 2009239471
(71) Applicant: Kaneka Corporation, Osaka-shi, Osaka 530-8288 (JP)
(72) Inventor: NISHIYAMA, Tozo, Takasago-shi Hyogo 676-8688 (JP); TAKAMI, Hiroyuki, Takasago-shi Hyogo 676-8688 (JP); NISHI, Teruyuki, Takasago-shi Hyogo 676-8688 (JP); TAKANO, Masayuki, Takasago-shi Hyogo 676-8688 (JP)
(74) Representative: Vossius & Partner
(86) International application number: PCT/JP2010/068206
(87) International publication number: WO 2011/046218

(57) **Abstract**

The present invention provides a transformant of Hansenula polymorpha that produces an antibody and/or a partial antibody, a transformant of Hansenula polymorpha, wherein an expression vector containing an antibody gene and/or a partial antibody gene is linearized, and the vector is incorporated into the chromosome, and a production method of an antibody and/or a partial antibody, including use of a transformant of Hansenula polymorpha.

## Description

### Technical Field

The present invention relates to Hansenula polymorpha that produces an antibody, a production method of an antibody using same, and an antibody obtained thereby.

### Background Art

In recent years, antibodies are widely used in the medical field of diagnosis, treatment and the like, with high affinity for antigen as characteristics. Recently, low molecular antibodies having smaller molecular weights by removing a part or the entirety of the region other than the antigen binding site of full length antibody are being developed. As the low molecular antibody, for example, Fab, scFv, F(ab')₂ and the like can be mentioned (non-patent document 1). The characteristic of the low molecular antibody is, for example, expected high accumulation in the target moiety.

Of those, a low molecular antibody expected to have utility as a next generation protein pharmaceutical product is Fab-type antibody. Fab-type antibody is a full length antibody less Fc region and hinge moiety, and consists of Fd chain and L chain. Fab-type antibody has been produced by a method including degrading full length antibody by digestion with an enzyme such as papain and the like, followed by purification. However, a gene recombination method including directly producing Fab-type antibody alone is considered at present (non-patent document 2).

In general, to produce a protein by a gene recombination method, a host appropriate for the protein needs to be selected, such as animal cell, insect such as Bombyx mori and the like and insect cell, animals such as chicken, bovine and the like, microorganisms such as Escherichia coli, yeast and the like.
In proteins having a high heteromeric structure such as antibody, since expression is reported only in a particular species of yeast (non-patent document 3), a particularly optimal species needs to be selected.

At present, antibody pharmaceutical products are produced by mainly using animal cells such as CHO (Chinese Hamster Ovary) cell and the like. However, the problem is that expensive media and long-time culture are required and the production cost becomes very high. While the production of a heterologous protein by Escherichia coli, which is most frequently used as a production host for biopharmaceuticals, is advantageous in that the proliferation rate of Escherichia coli is high and the production cost is low, the tendency of the absence of sugar chain modification, insufficient folding that causes accumulation of protein in an inactive form in bacterial cells and the like is often seen. In addition, it also has a demerit of complicated purification process, since Escherichia coli has difficulty in secretory expression of heterologous protein.

Thus, to establish an economical, practical and highly productive culture technique using yeast as an alternative host, several attempts have been reported to produce an antibody using Saccharomyces cerevisiae, or NRRL Y-11430 strain of Pichia pastoris, which is a methanol assimilatory yeast (non-patent document 3). Yeast enables a large-scale, high density culture in an economical medium, and can produce a heterologous protein at a low cost. Using signal peptide, secretory expression in a culture medium is possible and purification process can be facilitated. In addition, since it is a eucaryote, the merit of possible post-translational modification such as sugar chain addition and the like is also expected.

Hansenula polymorpha is known as a host for production of a heterologous protein, and examples of the production include green fluorescence protein, human serum albumin, Hepatitis B virus surface antigen, human epidermal growth factor, hirudin and the like (non-patent document 4). However, an example of the expression of an antibody at a practical expression level is not known.

While an attempted production of an antibody by introducing an expression vector in a circular state, which contains L chain and Fd chain genes of a mouse-derived Fab-type antibody, into Hansenula polymorpha has been reported, a culture supernatant showing independent secretory expression of L chain and Fd chain was only obtained, where most of the protein expressed was accumulated in the bacterial cell with L chain and Fd chain independently accumulated, and the expression of antibody has not been successful (non-patent document 5).

### [Document List]

### [non-patent documents]

non-patent document 1: Bio/Technology (1991) 9: 545-551
non-patent document 2: Science (1988) 240: 1041-1043
non-patent document 3: Biotechnol Lett (2007) 29: 201-212
non-patent document 4: Gellissen, G. (Ed.), Hansenula polymorpha Biology and Applications.,Chapter10 p147-155 (2002), Wiley-VCH (Weinheim, Germany)
non-patent document 5: Appl Microbiol Biotechnol. (2001) 56:157-64.

### [SUMMARY OF THE INVENTION]

### Problems to be Solved by the Invention

The problem of the present invention is secretory expression of an antibody, specifically a partial antibody, particularly a Fab-type antibody, by using Hansenula polymorpha. Moreover, the problem is provision of a Hansenula polymorpha transformant capable of secretory expression of an antibody, specifically a partial antibody, particularly a Fab-type antibody. In addition, the problem is provision of a method of producing the transformant, and a vector to be used for the method.

### Means of Solving the Problems

In the present invention, a transformant that expresses an antibody and a partial antibody, each in an activity form, has been successfully prepared by transforming Hansenula polymorpha by using a linearized vector. In addition, the present invention has succeeded in the secretory expression of an antibody and a partial antibody by culturing the transformant. Since a failure of secretory expression of a Fab-type antibody in Hansenula polymorpha using a circular expression vector is known, use of a linear expression vector was not expected to succeed. However, the use of a linear expression vector has unexpectedly solved the problem, which resulted in the completion of the present invention.

Accordingly, the present invention provides
[1] a transformant of Hansenula polymorpha that produces an antibody and/or a partial antibody;
[2] the transformant of the above-mentioned [1], comprising an expression vector comprising an antibody gene and/or a partial antibody gene incorporated in a linear state into a chromosome;
[3] the transformant of the above-mentioned [2], wherein the expression vector has a promoter and a base sequence encoding a signal sequence on the 5'-side, and a termination codon and/or a terminator sequence on the 3'-side, of the antibody gene and/or the partial antibody gene;
[4] the transformant of the above-mentioned [2] or [3], wherein the expression vector comprises a homology base sequence with not less than 50% of sequence identity with not less than 50 bp base sequence contained in the chromosome of Hansenula polymorpha;
[5] the transformant of the above-mentioned [4], wherein the homology base sequence is the promoter sequence present on the 5'-side of the antibody gene and/or the partial antibody gene, and/or the terminator sequence present on the 3'-side of the antibody gene and/or the partial antibody gene;
[6] the transformant of any one of the above-mentioned [2] to [5], wherein the number of copies of the expression vector per chromosome is not less than 2 copies;
[7] the transformant of any one of the above-mentioned [2] to [6], wherein the expression vector is incorporated into the chromosome in an MOX terminator region and/or an MOX promoter region on the chromosome;
[8] the transformant of any one of the above-mentioned [2] to [7], wherein the function of the MOX gene on the chromosome is partially or completely deficient;
[9] the transformant of any one of the above-mentioned [1] to [8], which is selected by using an auxotrophic complementary gene and/or a drug resistance gene as a selection marker;
[10] the transformant of the above-mentioned [9], wherein the auxotrophic complementary gene and/or the drug resistance gene are/is one or more genes selected from the group consisting of URA3, LEU2, ADE1, HIS4, G418 resistance gene, zeocin resistance gene and hygromycin resistance gene;
[11] the transformant of any one of the above-mentioned [2] to [10], wherein the partial antibody gene contained in the expression vector is a Fab-type antibody gene;
[12] the transformant of the above-mentioned [11], wherein the expression vector containing the Fab-type antibody gene is a base sequence containing an L chain gene and an Fd chain gene, a base sequence containing an L chain Fd chain fusion gene and/or a base sequence containing an Fd chain L chain fusion gene;
[13] the transformant of any one of the above-mentioned [1] to [12], which secretes an antibody and/or a partial antibody;
[14] the transformant of any one of the above-mentioned [1] to [13], wherein the antibody and/or the partial antibody are/is an antibody selected from a human antibody, a humanized antibody, a chimera antibody and a mouse antibody and/or a partial antibody derived therefrom;
[15] the transformant of any one of the above-mentioned [1] to [14], wherein the antibody and/or the partial antibody are/is bound to one or more antigens selected from the group consisting of CD20, HER2, IL2R, CD33, CD52, EGFR, VEGF, CD3, CD25, TNFα, CD11, IgE, CD2, α4integrin, CD80, CD86, IL6R, C5a, GPIIb/IIIa, RSVF Protein, VEGF-A and GM-CSF;
[16] the transformant of any one of the above-mentioned [13] to [15], wherein the partial antibody is a Fab-type antibody;
[17] the transformant of any one of the above-mentioned [13] to [16], wherein the secretory production amount of the Fab-type partial antibody in a culture supernatant is not less than 1 mg/mL when a Fab-type partial antibody derived from the antibody contained in HUMIRA (registered trade mark) is produced as the antibody and/or the partial antibody;
[18] a production method of an antibody and/or a partial antibody, comprising using the transformant of any one of the above-mentioned [1] to [17]; and
[19] an antibody and/or a partial antibody produced by the production method of the above-mentioned [18].

In addition, the present invention has one or plural characteristics below.

One of the characteristics of the present invention is a transformant of Hansenula polymorpha that produces an antibody and/or a partial antibody.

One of the characteristics of the present invention is a transformant of Hansenula polymorpha that secretes an antibody and/or a partial antibody, which is characterized by incorporation of a linearized expression vector containing an antibody gene and/or a partial antibody gene into a chromosome.

One of the characteristics of the present invention is a transformant of Hansenula polymorpha that secretes an antibody and/or a partial antibody, which is characterized in that the partial antibody is a Fab-type antibody.

One of the characteristics of the present invention is a production method of an antibody and/or a partial antibody, which uses a transformant of Hansenula polymorpha that produces the antibody and/or the partial antibody.

One of the characteristics of the present invention is an antibody and/or a partial antibody produced by a production method of an antibody and/or a partial antibody, which uses a transformant of Hansenula polymorpha that produces the antibody and/or the partial antibody.

### Effect of the Invention

According to the present invention, a transformant of Hansenula polymorpha that produces an antibody or a partial antibody, a production method of an antibody or a partial antibody using same, and an antibody or a partial antibody obtained thereby are provided.

### Brief Description of the Drawings

Fig. 1 relates to Western blotting in Example 8 of the present invention.
Fig. 2 relates to SDS-PAGE (CBB staining) in Example 10 of the present invention.

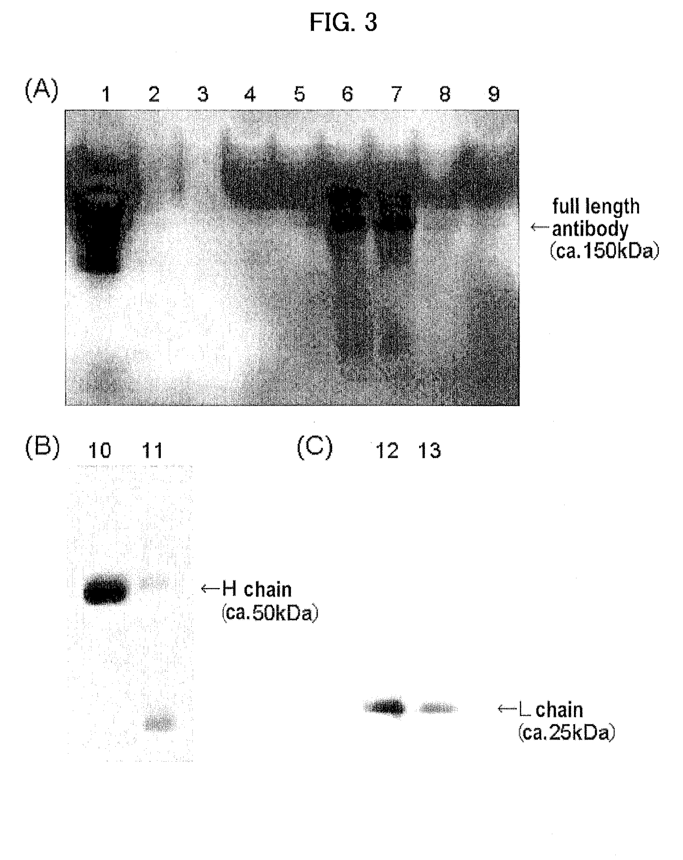

Fig. 3 relates to Western blotting in Example 13 of the present invention. (A) detection of eluted fraction and standard IgG by Anti-Human IgG-Fc HRP conjugate Antibody (manufactured by Bethyl Laboratories, Inc.) under non-reducing conditions, (B) detection of eluted fraction and standard IgG by Anti-Human IgG-Fc HRP conjugate Antibody (manufactured by Bethyl Laboratories, Inc.) under reducing conditions, (C) detection of eluted fraction and standard IgG by Anti-Human IgG (Fab SPECIFIC) HRP conjugate Antibody (manufactured by SIGMA) under reducing conditions. The samples were prepared as described in Example 12. Lane 1 shows standard IgG, lanes 2-9 show eluted fractions, lane 10 shows standard IgG, lane 11 shows eluted fraction with detection confirmed in (A) (sample of lane 6 in (A)), lane 12 shows standard IgG, lane 13 shows eluted fraction with detection confirmed in (A) (sample of lane 6 in (A)).

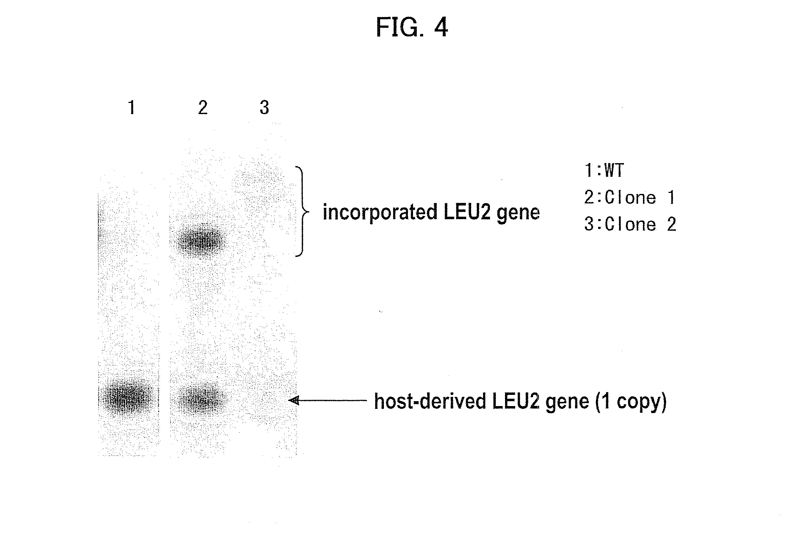

Fig. 4 relates to Southern blotting (BY5242 strain) in Example 14 of the present invention.

### [Description of Embodiments]

The present invention is explained in detail in the following by referring to the embodiments. The present invention is not limited by these.

In the present invention, the antibody refers to a heterotetrameric protein constituted by a disulfide bond of two polypeptide chains each of L chain and H chain, and is not particularly limited as long as it has an ability to bind to a particular antigen.

In the present invention, the partial antibody is a bindable fragment of the aforementioned antibody, which specifically refers to Fab-type antibody, F(ab')₂ type antibody, scFv type antibody, diabody type antibody, derivatives thereof and the like, and is not particularly limited as long as it has an ability to bind to a particular antigen.

In the present invention, the Fab-type antibody refers to a heteromeric protein wherein L chain and Fd chain of an antibody are bonded by an S-S bond, and is not particularly limited as long as it has an ability to bind to a particular antigen.

The F(ab')₂ type antibody refers to a heterotetrameric protein wherein Fd chains of two Fab-type antibodies are bonded by an S-S bond via a hinge moiety. The scFv type antibody refers to a protein wherein a heavy chain variable region (VH) and a light chain variable region (VL) are linked by a linker peptide to give a single strand, and the diabody type antibody refers to a dimer wherein two fragments (e.g., scFv etc.) of VH and VL bonded by a linker etc. are bonded. The derivatives thereof include those wherein the above-mentioned molecules are modified by a molecule having a protein stabilizing action such as polyethylene glycol (PEG) and the like.

While the kind of the antibody of the present invention is not particularly limited, for example, human antibody, humanized antibody, mouse antibody, dog antibody, cat antibody, horse antibody, bovine antibody, swine antibody, chicken antibody and chimera antibody which is a fused antibody thereof can be mentioned. While the kind of the partial antibody (e.g., Fab-type antibody) of the present invention is not particularly limited, for example, a partial antibody (e.g., Fab-type antibody) consisting of a partial sequence of human antibody, humanized antibody, mouse antibody, dog antibody, cat antibody, horse antibody, bovine antibody, swine antibody, chicken antibody or chimera antibody which is a fused antibody thereof and the like can be mentioned. As the partial antibody of the present invention is particularly preferably a Fab-type antibody.

In addition, while the antigen to which the antibody and partial antibody (Fab-type antibody etc.) of the present invention is bonded is not particularly limited, antigens known as a target of drug discovery such as CD20, HER2, IL2R, CD33, CD52, EGFR, VEGF, CD3, CD25, TNFα, CD11, IgE, CD2, α4integrin, CD80, CD86, IL6R, C5a, GPIIb/IIIa, RSVF Protein, VEGF-A, GM-CSF and the like can be preferably mentioned.

The gene encoding the antibody of the present invention is an L chain gene and an H chain gene (i.e., combination of L chain gene and H chain gene).

Of the genes encoding the partial antibody of the present invention, a gene encoding a Fab-type antibody may be L chain gene and Fd chain gene (i.e., combination of L chain gene and Fd chain gene), or L chain Fd chain fusion gene or Fd chain L chain fusion gene. The L chain Fd chain fusion gene is a gene having L chain gene and Fd chain gene in order, and a linker sequence between them (i.e., L chain gene - linker sequence - Fd chain gene from the upstream), and the Fd chain L chain fusion gene is a gene having Fd chain gene and L chain gene in order, and a linker sequence between them (i.e., Fd chain gene - linker sequence - L chain gene from the upstream). The preparation method of the genes to be used in the present invention such as L chain gene, H chain gene, Fd chain gene and the like is not particularly limited, and they may be prepared from various antibody-producing cells, hybridoma and phage display library by PCR and the like, or may be prepared by total synthesis of the gene from the base sequence.

In the present invention, Hansenula polymorpha is preferably a strain such as NCYC495 (ATCC14754), 8V (ATCC34438), DL-1 (ATCC26012) and the like. These strains are available from American Type Culture Collection. In addition, derivative strain and the like from the strain are also included and, for example, leucine requiring strains such as BY4329 derived from NCYC495, BY5242 derived from 8V, BY5243 derived from DL-1, and the like can be mentioned, which are available from National BioResource Project.

In the present invention, the transformant is a transformant of Hansenula polymorpha that produces an antibody and/or a partial antibody (Fab-type antibody etc.). The transformant can be obtained by transforming host Hansenula polymorpha with an expression vector containing a linearized antibody and/or a partial antibody (Fab-type antibody etc.) gene. Here, linearized state of a vector in the present invention means a state where one or more sites of a circular expression vector is/are cleaved to become linear. As a method of preparing a linear vector, a circular vector may be linearized by cleaving with a restriction enzyme etc. or by a method including conducting PCR using a circular vector as a template.

The expression vector to be used in the present invention is not particularly limited as long as it can express an antibody and/or a partial antibody in host Hansenula polymorpha. Examples of usable expression vector include pSH19, pSH15, pUC19 and the like. The expression vector to be used in the present invention contains the aforementioned antibody and/or partial antibody (Fab-type antibody etc.) gene in an expressible manner when incorporated into the chromosome of the host Hansenula polymorpha.

In the present invention, the expression vector of the Fab-type antibody contains a base sequence containing L chain gene and Fd chain gene, a base sequence containing L chain Fd chain fusion gene and/or a base sequence containing Fd chain L chain fusion gene. In the present invention, an expression vector of Fab-type antibody may be one kind of (single) vector wherein L chain gene and Fd chain gene are present on the same vector, as shown in Example 1, or two kinds of vectors wherein L chain gene and Fd chain gene are present on different vectors (i.e., combination of two kinds of expression vectors) as shown in Example 3. The same applies to the expression vector of antibody and expression vector of partial antibody other than Fab-type antibody.

In addition, the expression vector preferably has a promoter and a base sequence encoding a signal sequence on the 5'-side, and a termination codon and/or a terminator sequence on the 3'-side, of the antibody and/or partial antibody gene. Here, the promoter used in the present invention is not particularly limited as long as it permits induction of expression in Hansenula polymorpha and, for example, methanol oxidase (hereinafter to be abbreviated as MOX), GAP, TEF, FMD promoters and the like can be mentioned. Preferably, it is an MOX promoter or GAP promoter of Hansenula polymorpha (preferably host Hansenula polymorpha).

While the base sequence encoding a signal sequence is not particularly limited as long as it is a base sequence encoding a signal sequence that permits secretory expression of a protein in Hansenula polymorpha, and a base sequence encoding Mating Factor α(MFα) preprosignal (e.g., SEQ ID NO: 8) of Saccharomyces cerevisiae and the like can be mentioned.

Moreover, a base sequence encoding an appropriate linker sequence may be present between a base sequence encoding a signal sequence and an antibody gene and/or a partial antibody gene (e.g., L chain gene, Fd chain gene, H chain gene, L chain Fd chain fusion gene, Fd chain L chain fusion gene etc.). While the base sequence encoding a linker sequence is not particularly limited, it is preferably a base sequence capable of separating a signal sequence and an antibody and/or partial antibody gene product (L chain, Fd chain, H chain, L chain Fd chain fusion protein or Fd chain L chain fusion protein etc.) after secretory production. For example, a base sequence encoding the amino acid sequence of Lys-Arg, which is recognized and cleaved by KEX2p of Hansenula polymorpha, which is one kind of protease, can be used as a base sequence encoding a linker sequence.

The terminator sequence is not particularly limited as long as it enables Hansenula polymorpha to terminate the gene transcription. For example, a terminator of the MOX gene of Hansenula polymorpha can be used.

Moreover, the expression vector used in the present invention preferably has a base sequence having not less than 50% (preferably not less than 70%, more preferably not less than 80%, still more preferably not less than 90%, most preferably 100%) of sequence identity with not less than 50 bp base sequence contained in the chromosome of Hansenula polymorpha (preferably, host Hansenula polymorpha).

As used herein, "identity" means the proportion (%) of the same base sequences to all overlapping base sequences in the optimal alignment where two base sequences are aligned using a mathematic algorithm known in the relevant technical field (preferably, the algorithm is such that a gap can be introduced into one or both of the sequences for the optimal alignment). The identity of the base sequence in the present specification can be calculated using a homology calculation algorithm NCBI BLAST-2 (National Center for Biotechnology Information Basic Local Alignment Search Tool) and under the following conditions (gap open=5; gap extension=2; x_drop off=50; expectancy =10; filtering=ON)). Algorithms to determine the identity of a base sequences include, for example, the algorithm described in Karlin et al., Proc. Natl. Acad. Sci. USA, 90: 5873-5877 (1993) [the algorithm is incorporated in the NBLAST and XBLAST programs (version 2.0) (Altschul et al., Nucleic Acids Res., 25: 3389-3402 (1997))], the algorithm described in Needleman et al., J. Mol. Biol., 48: 444-453 (1970) [the algorithm is incorporated in the GAP program in the GCG software package], the algorithm described in Myers and Miller, CABIOS, 4: 11-17 (1988) [the algorithm is incorporated in the ALIGN program (version 2.0), which is part of the CGC sequence alignment software package], the algorithm described in Pearson et al., Proc. Natl. Acad. Sci. USA, 85: 2444-2448 (1988) [the algorithm is incorporated in the FASTA program in the GCG software package] and the like, and they can also be preferably used in the same manner.

In the expression vector, the base sequence having not less than 50% of sequence identity with not less than 50 bp base sequence contained in the chromosome of the aforementioned Hansenula polymorpha (preferably, host Hansenula polymorpha) is a promoter sequence present on the 5'-side of the antibody and/or partial antibody gene, and/or a termination sequence present on the 3'-side of the antibody and/or partial antibody gene. From such aspect, in a preferable embodiment, the expression vector used in the present invention has a promoter sequence of MOX gene or GAP gene of Hansenula polymorpha (preferably, host Hansenula polymorpha) as the promoter sequence present on the 5'-side of the antibody gene and/or the partial antibody gene, and/or a terminator sequence of MOX gene of Hansenula polymorpha (preferably, host Hansenula polymorpha) as the terminator sequence present on the 3'-side of the antibody gene and/or the partial antibody gene.

Preferable examples of the order of respective constituent elements contained in the expression vector of Fab-type antibody and the expression vector of antibody used in the present invention from the 5'-side to the 3'-side are as follows.
(1) first promoter sequence - first signal sequence - L chain gene - second promoter sequence - second signal sequence - Fd chain gene - terminator sequence
(2) first promoter sequence - first signal sequence - Fd chain gene - second promoter sequence - second signal sequence - L chain gene - terminator sequence
(3) first promoter sequence - first signal sequence - L chain gene - second promoter sequence - second signal sequence - H chain gene - terminator sequence
(4) first promoter sequence - first signal sequence - H chain gene - second promoter sequence - second signal sequence - L chain gene - terminator sequence
(5) combination of an expression vector containing (first promoter sequence - first signal sequence - L chain gene - first terminator sequence) and an expression vector containing (second promoter sequence - second signal sequence - Fd chain gene - second terminator sequence)
(6) combination of an expression vector containing (first promoter sequence - first signal sequence - L chain gene - first terminator sequence) and an expression vector containing (second promoter sequence - second signal sequence - H chain gene - second terminator sequence)

In (1) - (6), the first promoter and the second promoter may be the same or different. The first and the second promoters are preferably MOX promoter or GAP promoter of Hansenula polymorpha (preferably, host Hansenula polymorpha).

In (1) - (6), the first signal sequence and the second signal sequence may be the same or different. The first and the second signal sequences are preferably Mating Factor α (MFα) preprosignal of Saccharomyces cerevisiae.

In (5) and (6), the first terminator sequence and the second terminator sequence may be the same or different. The first and the second terminator sequence are preferably terminator sequences of MOX gene of Hansenula polymorpha.

The expression vector to be used in the present invention preferably includes selection markers such as auxotrophic complementary gene, drug resistance gene and the like. The transformant of the present invention can be obtained by transforming host Hansenula polymorpha with the above-mentioned expression vector, and selecting the transformant incorporating the expression vector by utilizing the selection marker. When the selection marker is not particularly limited, an auxotrophic complementary gene such as URA3 gene, LEU2 gene, ADE1 gene, HIS4 gene and the like, the transformant can be selected by recovery of a prototrophic strain phenotype in auxotrophic strains such as uracil, leucine, adenine and histidine. In the case of drug resistance genes such as G418 resistance gene, zeocin resistance gene, hygromycin resistance gene, and the like, the transformant can be selected by resistance on a medium containing G418, zeocin, hygromycin, respectively.

The position of the selection marker in an expression vector is not particularly limited as long as a transformant incorporating the expression vector can be selected using the selection marker. Preferably, it is on the 5'-side from the promoter on the 5'-side of an antibody gene and/or a partial antibody gene. In the above-mentioned embodiments (1) - (4), the 5'-side of the first promoter is preferable. In the embodiments (5) and (6), a selection marker is preferably present at the 5'-side of each of the first promoter and the second promoter.

Hansenula polymorpha can be transformed by a known method (e.g., electroporation etc.). For example, it can be transformed according to the method described in Meth. Enzymol., 194: 182-187 (1991), Proc. Natl. Acad. Sci. USA, 75: 1929 (1978) and the like.

In the transformant of the present invention, a linearized expression vector containing an antibody gene and/or a partial antibody (Fab-type antibody etc.) gene is preferably incorporated into the chromosome of host Hansenula polymorpha. While the number of copies of the expression vector per chromosome is not particularly limited and 1 copy or 2 or more copies. For high production, 2 or more copies are preferable. The number of copies of the expression vector per chromosome can be confirmed by, for example, Southern blotting method. To be specific, a genomic DNA is extracted from a transformant and treated with an appropriate restriction enzyme. Then, the treated genomic DNA is separated by agarose gel electrophoresis, transferred on a nylon membrane, and hybridized using a probe having a base sequence complementary to a suitable gene in an expression vector, whereby the number of copies of the expression vectors incorporated into the genomic DNA can be confirmed. Such confirmation method can be analyzed by, for example, the method shown in Example 14. As a method of efficiently obtaining a transformant with two or more copy number of an expression vector per chromosome, use of various selection marker genes (LEU2, URA3, ADE1, HIS4) with a transcription amount decreased by shortening the promoter region as a selection marker can be mentioned. To be specific, a method using LEU2 gene, URSA3 gene and the like with a shorter promoter region, as constructed in Example 3, as a selection marker can be mentioned. When a transformant incorporating two or more copies of expression vector is incorporated, a plurality of expression vectors may be incorporated into the same site, or one copy each at different sites.

The method of incorporating an expression vector containing an antibody gene and/or a partial antibody (Fab-type antibody etc.) gene on the chromosome may be a nonspecific one without using a homologous region, one using one homologous region, or a double integration type using two homologous regions. The homologous region only needs to show not less than 50% of sequence identity when compared with the base sequences of the expression vector and chromosome. The length thereof is not particularly limited, either, but not less than 50 bp is preferable. The sequence identity of a homologous region is more preferably not less than 70%, further preferably not less than 80%, still more preferably not less than 90%, and most preferably 100%. Here, the "identity" is determined as mentioned above.

It is known that homologous recombination is preferably performed by increasing the sequence identity of the base sequence of the terminal portion of a homologous region, and those of ordinary skill in the art can select a homologous region having a preferable sequence identity. The length of the homologous region is more preferably not less than 100 bp, further preferably not less than 500 bp, and still more preferably not less than 1000 bp. In addition, not more than 10 kbp is more preferable, not more than 5 kbp is further preferable, and not more than 3 kbp is still more preferable. When it is too long, the size of the vector increases and incorporation of the vector becomes difficult, which is well known to those of ordinary skill in the art. The upper limit can be appropriately determined. It is known that homologous recombination is preferably performed by increasing the length of the homologous region, and the lower limit can be appropriately selected. Those of ordinary skill in the art can select a homologous region with a preferable length. For incorporation utilizing a homologous region, for example, a homologous region of a plasmid vector is cleaved with restriction enzyme at one or more sites such that the homologous region comes to the terminal of the linear expression vector, and transformation can be performed.

Accordingly, as mentioned above, when the expression vector to be used in the present invention has a base sequence having not less than 50% (preferably not less than 70%, more preferably not less than 80%, further preferably not less than 90%, most preferably 100%) of sequence identity with the base sequence of not less than 50 bp contained in the chromosome of Hansenula polymorpha (preferably, host Hansenula polymorpha), particularly when the base sequence having not less than 50% of sequence identity with the base sequence of not less than 50 bp contained in the chromosome of Hansenula polymorpha (preferably, host Hansenula polymorpha) is a promoter sequence present on the 5'-side of an antibody gene and/or a partial antibody gene and/or a terminator sequence present on the 3'-side of an antibody gene and/or a partial antibody gene, incorporation onto the chromosome by homologous recombination is expected to be facilitated.

In addition, while the site of incorporation of an expression vector containing an antibody gene and/or a partial antibody (Fab-type antibody etc.) gene on the chromosome of Hansenula polymorpha is not particularly limited as long as the transformant produces the antibody and/or the partial antibody (Fab-type antibody etc.), it is preferably around MOX gene, more preferably MOX promoter region or an MOX terminator region of MOX gene. For example, for homologous integration into MOX terminator, an expression vector containing MOX terminator, which is linearized by cleaving with the EcoRV site present at one site in the MOX terminator sequence, is used for incorporation into the MOX terminator.

Incorporation into the vicinity of MOX gene is expected to provide an effect of easy expression of an antibody gene and increased production amount.

Using, as the base sequence of the promoter at the 5'-side of an antibody gene and/or a partial antibody gene contained in the expression vector used in the present invention, and/or the base sequence of the terminator at the 3'-side, a base sequence having sequence identity of not less than 50% (preferably not less than 70%, more preferably not less than 80%, further preferably not less than 90%, most preferably 100%) with an MOX promoter region sequence (e.g., SEQ ID NO: 1) and an MOX terminator region sequence (SEQ ID NO: 2) on the chromosome of Hansenula polymorpha, a transformant wherein a homologous recombination occurs in an MOX terminator region and/or an MOX promoter region, and the incorporation site is an MOX terminator region and/or an MOX promoter region on the chromosome, can be obtained.

The MOX gene deficiency refers to the state where a part or the entirety of the MOX gene on the chromosome does not function due to deficiency or destruction and the like. The deficiency of MOX gene can be confirmed by, as in Example 6, the presence or absence of growth in a medium containing methanol as a sole carbon source. As a preparation method of a strain deficient in MOX gene, a method including, as in Example 6, inserting a Fab-type antibody expression vector into MOX gene and the like can be mentioned.

The state where a part or the entirety of the MOX gene on the chromosome does not function due to deficiency or destruction and the like refers to (1) a state where transcription regulation of promoter, operator and the like of MOX gene does not function, and MOX gene is not expressed, (2) a state where frame shift occurs, and MOX protein is not expressed as active protein, (3) a state where a part or the entirety of structural gene in MOX gene is deficient and active protein is not expressed, and the like, which are due to the deficiency of base in the base sequence of gene on the chromosomal DNA. When MOX gene is deficient due to homologous recombination, the state of (3) can be mentioned. For example, as mentioned above, when an expression vector is incorporated into an MOX promoter region and an MOX terminator region of an MOX gene, the deficiency of the MOX gene is expected.

Being MOX gene deficient, the methanol metabolism of the transformant decreases, which is expected to provide an effect that efficient methanol induction is possible.

The transformant obtained as mentioned above can be cultured by a known method. For example, as a method of producing an antibody and a partial antibody (Fab-type antibody etc.) by using the transformant of the present invention, a method including culturing the above-mentioned transformant of Hansenula polymorpha and accumulating the (partial) antibody in the culture supernatant thereof can be mentioned. For culture of the transformant, any medium can be used as long as it contains a nutrient source generally utilizable for Hansenula polymorpha. For example, a conventional medium containing an appropriate mixture and blend of nutrient sources such as carbon sources (e.g., saccharides such as glucose, sucrose, maltose and the like, organic acids such as lactic acid, acetic acid, citric acid, propionic acid and the like, alcohols such as methanol, ethanol, glycerol and the like, hydrocarbons such as paraffin and the like, fats and oils such as soybean oil, rape seed oil and the like, mixtures thereof and the like), nitrogen sources (e.g., ammonium sulfate, ammonium phosphate, urea, yeast extract, meat extract, peptone, corn steep liquor and the like), other inorganic salts, vitamins and the like can be used, and both batch culture and continuous culture can be employed. It is also possible to induce expression of antibody gene and the like during culture. For example, when expression of antibody gene and the like is induced with MOX promoter, methanol and the like is added during culture. In the present invention, culture can be generally performed under normal conditions, for example, by cultivating at pH 2.5 - 10.0, temperature range 10°C - 48°C aerobically for 10 hr - 10 days.

A transformant that produces not less than 1 mg/L of Fab-type antibody secretory production amount in the culture supernatant when Fab-type antibody wherein L chain and Fd chain of HUMIRA (registered trade mark) are bonded by an S-S bond in the present invention is subjected to secretory production by a test tube culture is a transformant of Hansenula polymorpha that produces Fab-type antibody at a concentration of not less than 1 mg/L by transforming Hansenula polymorpha by the method shown in Example 5 with an expression vector containing Fab-type antibody gene shown in Example 1, culturing the obtained transformant in a test tube by the method shown in Example 7, and analyzing the secretory production amount of Fab-type antibody in the obtained culture supernatant.

The antibody and/or partial antibody (Fab-type antibody etc.) produced by the transformant of the present invention may be in the state of a culture supernatant, or may be isolated therefrom by any method. The antibody and/or partial antibody (Fab-type antibody etc.) can be isolated from a culture supernatant by using known protein purification methods in an appropriately combination. For example, the isolation can be performed as follows. First, the transformant is cultured in a suitable medium, and fungus is removed from the culture supernatant by centrifugation or filtration treatment of the culture medium. The obtained culture supernatant is subjected to methods such as salting out (ammonium sulfate precipitation, sodium phosphate precipitation and the like), solvent precipitation (protein fraction precipitation method by acetone, ethanol and the like), dialysis, gel filtration chromatography, ion exchange chromatography, hydrophobic chromatography, affinity chromatography, reversed-phase chromatography, ultrafiltration and the like alone or in combination, whereby the antibody and/or partial antibody (Fab-type antibody etc.) of the present invention is isolated from the culture supernatant.

While the isolated antibody and/or partial antibody (Fab-type antibody etc.) can be directly used, or can also be used after modification that provides pharmacological changes such as pegylation and the like, modification to add a function of enzyme, isotope and the like. In addition, various formulation treatments may be applied.

### Examples

The present invention is explained in detail in the following by referring to Examples, which are not to be construed as limitative. The detailed operation methods and the like relating to recombinant DNA technique used in the following Examples are described in the following book: Molecular Cloning 2nd Edition (Cold Spring Harbor Laboratory Press, 1989), Current Protocols in Molecular Biology (Greene Publishing Associates and Wiley-Interscience).

MOX promoter (SEQ ID NO: 1), MOX terminator (SEQ ID NO: 2), LEU2 gene (SEQ ID NO: 3), LEU2 gene with shortened promoter region (SEQ ID NO: 4), URA3 gene (SEQ ID NO: 5), URA3 gene with shortened promoter region (SEQ ID NO: 6) and GAP promoter (SEQ ID NO: 7), which were utilized in the construction of antibody expression vectors, were prepared by PCR using genomic DNA of Hansenula polymorpha 8V strain as a template. Mating Factor α preprosignal (MFα, SEQ ID NO: 8) was prepared by PCR using genomic-DNA of Saccharomyces cerevisiae S288c strain as a template. An antibody gene was prepared by PCR using chemically synthesized L chain (SEQ ID NO: 9) and H chain (SEQ ID NO: 10) (JP-A-2009-082033) as a template based on the published sequence information of complete humanized anti TNF-α antibody (adalimumab; HUMIRA (registered trade mark)).

Prime STAR HS DNA Polymerase (manufactured by TAKARA BIO INC.) was used for PCR, and the reaction conditions were those of the method described in the attached manual. Genomic DNA was prepared by using *Gentorukun* (manufactured by TAKARA BIO INC.) from each strain under the conditions described therein.

Each plasmid was prepared by transforming Escherichia coli DH5α competent cell (manufactured by TAKARA BIO INC.). The plasmid was prepared from plasmid-retaining strain by using QIAprep spin miniprep kit (manufactured by QIAGEN).

### (Example 1) Construction of Fab antibody expression vector -1

A gene segment (SEQ ID NO: 11) having HindIII-NotI-BamHI-SpeI-MunI-BglII-XbaI-EcoRI site was totally synthesized, and this was inserted into pUC19 HindIII-EcoRI site to prepare pUC-1. A gene segment having HindIII site on both sides of LEU2 gene was prepared by PCR using primers 1 and 2 (SEQ ID NOs: 12 and 13) and, after HindIII treatment, inserted into pUC-1 HindIII site (pUC-2). Then, a gene segment having BamHI site on both sides of MOX promoter was prepared by PCR using primers 3 and 4 (SEQ ID NOs: 14 and 15) and, after BamHI treatment, inserted into pUC-2 BamHI site (pUC-3). A gene segment having MunI site on both sides of MOX promoter was prepared by PCR using primers 5 and 6 (SEQ ID NOs: 16 and 17) inserted into pUC-3 MunI site after MunI treatment (pUC-4). A gene segment having XbaI site on both sides of MOX terminator was prepared by PCR using primers 7 and 8 (SEQ ID NOs: 18 and 19) and, after XbaI treatment, inserted into pUC-4 XbaI site (pUC-PmPmTm).

A gene segment having SpeI site at the upstream of MFα was prepared by PCR using primers 9 and 10 (SEQ ID NOs: 20 and 21). A gene segment having 20 bp MFα 3' terminal at the upstream of L chain and SpeI site at the downstream thereof was prepared by PCR using primers 11 and 12 (SEQ ID NOs: 22 and 23). These gene segments were mixed and used as a template, and a gene segment having SpeI site on both sides of a fusion gene of MFα and L chain was prepared by PCR using primers 9 and 12. After SpeI treatment, this gene segment was inserted into pUC-PmPmTm SpeI site, and pUC-PmLPmTm was constructed. A gene segment having BglII site at the upstream of MFα was prepared by PCR using primers 13 (SEQ ID NO: 24) and 10. A gene segment having 20 bp MFα 3' terminal at the upstream of Fd chain and BglII site at the downstream thereof was prepared by PCR using primers 14 and 15 (SEQ ID NOs: 25 and 26). These gene segments were mixed and used as a template, and a gene segment having BglII site on both sides of a fusion gene of MFα and Fd chain was prepared by PCR using primers 13 and 15. After BglII treatment, this gene segment was inserted into pUC-PmLPmTm BglII site, and pUC-PmLPmFTm was constructed. This expression vector is designed such that L chain and Fd chain of Fab-type antibody are each expressed under the control of different MOX promoters.

### (Example 2) Construction of Fab antibody expression vector -2

The expression vector was constructed such that L chain and Fd chain of Fab-type antibody were expressed under the control of GAP promoter. That is, a gene segment having BamHI site on both sides of GAP promoter was prepared by PCR using primers 16 and 17 (SEQ ID NOs: 27 and 28) and, after BamHI treatment, inserted into the pUC-2 BamHI site described in Example 1 (pUC-5). A gene segment having MunI site on both sides of GAP promoter was prepared by PCR using primers 18 and 19 (SEQ ID NOs: 29 and 30) and, after MunI treatment, inserted into pUC-5 MunI site (pUC-6). A gene segment having XbaI site on both sides of the MOX terminator prepared in Example 1 was inserted into pUC-6 XbaI site (pUC-PgPgTm). A gene segment having SpeI site on both sides of a fusion gene of MFα and L chain prepared in Example 1 was inserted into pUC-PgPgTm SpeI site, and pUC-PgLPgTm was constructed. Then, a gene segment having BglII site on both sides of fusion gene of MFα and Fd chain prepared in Example 1 was inserted into pUC-PgLPgTm BglII site, and pUC-PgLPgFTm was constructed. This expression vector is designed such that L chain and Fd chain of Fab-type antibody are each secreted to express under the control of different GAP promoters.

### (Example 3) Construction of Fab-type antibody each chain (L chain, Fd chain) expression vector

A fragment having MunI site on both sides of the MOX promoter prepared in Example 1 was prepared by PCR and, after MunI treatment, inserted into the pUC-1 MunI site constructed in Example 1 (pUC-7). A gene segment having XbaI site on both sides of the MOX terminator prepared in Example 1 was prepared by PCR and inserted into pUC-7 XbaI site (pUC-8).

In L chain expression vector, gene segments having HindIII site on both sides of LEU2 gene and LEU2 gene with shortened promoter region were prepared by PCR using primers 1 and 2, primer 20 (SEQ ID NO: 31) and 2 and, after HindIII treatment, inserted into pUC-8 HindIII site, respectively (pUC-PmTm-1, pUC-PmTm-2). A gene segment having BglII site at the upstream of MFα was prepared by PCR using primers 13 and 10. A gene segment having 20 bp MFα 3' terminal at the upstream of L chain and BglII site at the downstream thereof was prepared by PCR using primers 11 and 21 (SEQ ID NOs: 32). These gene segments were mixed and used as a template, and a gene segment having BglII site on the both sides of a fusion gene of MFα and L chain was prepared by PCR using primers 13 and 21. After BglII treatment, this gene segment was inserted into BglII sites of pUC-PmTm-1 and pUC-PmTm-2, and pUC-PmLTm-1 and pUC-PmLTm-2 were constructed. This L chain expression vector is designed such that L chain gene of Fab-type antibody is expressed by secretion under the control of MOX promoter. In addition, pUC-PmLTm-1 uses LEU2 gene as a selection marker, and pUC-PmLTm-2 uses LEU2 gene with shortened promoter region as a selection marker.

In Fd chain expression vector, gene segments having EcoRI site on both sides of URA3 gene and URA3 gene with shortened promoter region were prepared by PCR using primers 22 and 23 (SEQ ID NOs: 33 and 34), primer 24 (SEQ ID NO: 35) and 23 and, after EcoRI treatment, inserted into EcoRI site of pUC-8 (pUC-PmTm-3, pUC-PmTm-4). A gene segment having BglII site on both sides of a fusion gene of MFα and Fd chain prepared in Example 1 was inserted into BglII site of each of pUC-PmTm-3 and pUC-PmTm-4, and pUC-PmFTm-1 and pUC-PmFTm-2 was constructed. These Fd chain expression vectors are designed such that Fd chain gene of Fab-type antibody is expressed by secretion under the control of MOX promoter. In addition, pUC-PmFTm-1 uses URA3 gene as a selection marker and pUC-PmFTm-2 uses URA3 gene with shortened promoter region as a selection marker.

### (Example 4) Construction of full length antibody expression vector

A gene segment having BglII site at the upstream of MFα prepared in Example 1 was prepared by PCR. A gene segment having 20 bp 3' terminal MFα at the upstream of H chain, and BglII site at the downstream thereof was prepared by PCR using primers 14 and 25 (SEQ ID NO: 36). These gene segments were mixed and used as a template, and a gene segment having BglII site on both sides of a fusion gene of MFα and H chain was prepared by PCR using primers 13 and 25. After BglII treatment, this gene segment was inserted into BglII site of pUC-PmLPmTm prepared in Example 1 and pUC-PmLPmHTm was constructed. This expression vector is designed such that L chain and H chain of Fab-type antibody are each expressed by secretion under the control of different MOX promoters.

### (Example 5) Obtainment of recombinant antibody expression strain by transformation of Hansenula polymorpha

Various antibody expression vectors constructed in Examples 1 - 4 were linearized by digesting with EcoRV or NruI site in MOX terminator. Using the fragments, Hansenula polymorpha was transformed. The transformation method included inoculating each of Hansenula polymorpha BY4329 (NCYC495-derived, leu1-1), BY5242 (8V-derived, leu1-1), BY5243 (DL-1-derived, leu1-1) and BY4868 (NCYC495-derived, ura3 leu1-1) in 3 ml of YPD medium (1% yeast extract bacto (manufactured by Difco), 2% tryptone bacto (manufactured by Difco), 2% glucose), and culturing with shaking at 37°C overnight to give preculture medium. The obtained preculture medium (500 µl) was inoculated in 50 ml of YPD medium, and cultured with shaking at 30°C until OD600 reached 1 - 1.5 and collected (3000×g, 10 min, 20°C). The fungus was suspended in 10 ml of 50 mM potassium phosphate buffer, pH 7.5 containing 250 µl of 1M DTT (final concentration 25 mM), and the suspension was incubated at 37°C for 15 min. After harvest (3000×g, 10 min, 4°C), the fungus was re-suspended in 50 ml of ice-cooled STM buffer (270 mM sucrose, 10 mM Tris-HCl, 1 mM magnesium chloride, pH 7.5). After harvest (3000×g, 10 min, 4°C), the fungus was re-suspended in 25 ml of ice-cooled STM buffer. After harvest (3000×g, 10 min, 4°C), the fungus was re-suspended in 250 µl of ice-cooled STM buffer and used as a competent cell solution. The competent cell solution (60 µl) and each linear plasmid solution (3 µl) (DNA amount 0.5 - 1 µg) were mixed, placed in an electroporation cuvette (disposable cuvette electrode, electrode interval 2 mm; manufactured by BM EQUIPMENT), and electroporated under the conditions of 7.5 kV/cm, 10 µF, 900 Q. Thereafter, the fungus was suspended in YPD medium (1 ml) and stood at 37°C for 1 hr. After harvest (3000×g, 5 min, room temperature), the fungus was washed with 1 ml of YNB medium (0.67% yeast nitrogen base (manufactured by Difco), 1% glucose), and harvested (3000×g, 5 min, room temperature) again. The fungus was suspended in an adequate amount of YNB medium, and smeared on a YNB selection agar plate. Strains that grew by static culture at 37°C for 3 days were selected and various recombinant antibody expression strains were obtained.

### (Example 6) Obtainment of Fab-type antibody expression strain deficient in MOX gene on chromosome

A Fab-type antibody expression strain was obtained by the method of Example 5, the obtained transformant was inoculated in a medium containing methanol as a single carbon source (0.67% yeast nitrogen base (manufactured by Difco), 1% methanol), and cultured with shaking at 30°C for 72 hr. A strain that did not grow was selected to give Fab-type antibody expression strain deficient in the MOX gene on the chromosome.

### (Example 7) Antibody secretion in culture supernatant and measurement of antibody secretion by ELISA method

The amount of expression by secretion of Fab antibody and complete antibody in culture supernatant was analyzed by sandwich ELISA (Enzyme-Linked Immunosorbent Assay) method.

Culture supernatant was prepared as follows. That is, each antibody expression strain obtained in Example 5 or 6 was inoculated in 2 ml of BMGMY medium (1% yeast extract bacto, 2% peptone, 1.34% yeast nitrogen base, 0.4 mg/l Biotin, 100 mM potassium phosphate (pH 6.0), 1% Glycerol, 1% Methanol), cultured by shaking at 30°C for 72 hr, and centrifuged (15,000 rpm, 1 min, 4°C) to give a culture supernatant.

In sandwich ELISA, ANTI-HUMAN IgG (Fab SPECIFIC) Affinity Isolated Antigen Specific Antibody (manufactured by SIGMA) 5000-fold diluted with immobilization buffer (0.1N sodium bicarbonate solution, pH 9.6) was added to a plate for ELISA (MaxiSoap; manufactured by NUNC) at 50 µl/well, and incubated overnight at 4°C. After incubation, the solution in the well was removed, 5-fold diluted IMMUNOBLOCK (manufactured by Dainippon Sumitomo Pharmaceuticals Co., Ltd.) was added at 250 µl/well, and the mixture was stood at room temperature for 1 hr for blocking. Each well was washed 3 times with PBST (PBS (manufactured by TAKARA BIO INC.)+0.1% Tween20), serially diluted standard protein (Anti-Human IgG Fab; manufactured by rockland) and a diluted solution of culture supernatant were added at 50 µl/well, and the mixture was reacted at room temperature for 1 hr. The solution in the well was removed, and the well was washed twice with PBST, the secondary antibody solution (secondary antibody: Anti-Human IgG (Fab SPECIFIC) PEROXIDASE CONJUGATE Antibody developed in Goat Affinity Isolated Antibody; manufactured by SIGMA) 8000-fold diluted with PBSTIB (PBST+1/50 dilution IMMUNOBLOCK) solution was added at 50 µl/well, and the mixture was reacted at room temperature for 1 hr. The solution in the well was removed, and the well was washed 4 times with PBST, TMB 1-Component Microwell Peroxidase Substrate, SureBlue (manufactured by KPL) was added at 100 µl/well, and the mixture was stood at room temperature for 20 min. TMB Stop Solution (manufactured by KPL) was added at 100 µl/well to quench the reaction, and the absorbance at 450 nm was measured by a microplatereader (BenchMark Plus; manufactured by Bio-Rad). The antibody in the culture supernatant was quantified by using a standard protein analytical curve. The accumulation of the antibody expressed by secretion in the culture supernatant was confirmed by this method (Table 1).

**Table 1**

| Host | vector | number copy on chromosome | MOX gene | clone No. | Fab (mg/L) |
|---|---|---|---|---|---|
| BY4329 | pUC-PmLPmFTm | 1 | not defective | 1 | 7.6 |
| | | 1 | not defective | 2 | 6.4 |
| | pUC-PmLPmFTm | 1 | defective | 1 | 9.8 |
| | pUC-PgLPgFTm | 1 | not defective | 1 | 10.1 |
| BY5242 | pUC-PmLPmFTm | 1 | not defective | 1 | 4.6 |
| | | 1 | not defective | 2 | 4.1 |
| BY5243 | pUC-PmLPmFTm | 1 | not defective | 1 | 2.1 |
| | | 1 | not defective | 2 | 4.8 |
| BY4868 | pUC-PmLPmLTm-1 | 1 | not defective | 1 | 6.5 |
| | pUC-PmLPmFTm-1 | 1 | | | |
| | pUC-PmLPmLTm-2 | 2 | not defective | 1 | 10.8 |
| | pUC-PmLPmFTm-2 | 2 | | | |

### (Example 8) Detection of Fab antibody by Western blotting

Fab antibody and complete antibody were detected by the Western blotting method. That is, the culture supernatant obtained in Example 5 and fungus fraction (10 µl) were each mixed with 2×sample buffer (10% SDS, 50% Glycerol, 0.3125M Tris-HCl, pH 6.8, 0.01% BPB) (10 µl) and subjected to SDS-PAGE using 15% e-PAGEL (manufactured by ATTO) under non-reducing conditions. Then, two sheets of filter paper immersed in anode buffer 1 (0.3M Tris, 10% Methanol, pH 10.4), one sheet of filter paper and one sheet of PVDF membrane (Immobilon-P; manufactured by Millipore) immersed in anode buffer 2 (25 mM Tris, 10% Methanol, pH 10.4), and the gel after electrophoresis and three sheets of filter paper immersed in cathode buffer (25 mM Tris, 192 mM glycine, 10% Methanol, pH 9.4) for 15 min were placed in this order on a semi-dry blotting apparatus (MilliBlot-Graphite Electroblotter I; manufactured by Millipore), and constant electric current of 120 mA was flown for 90 min to transfer the protein on the gel to the PVDF membrane. The membrane after transfer was blocked by immersing in TBST (0.24% Tris, 0.8% NaCl, pH 7.6, 0.1% Tween20) containing 3% BSA for 1 hr. The membrane was washed with TBST 15 min×1, 5 minx2 and reacted with secondary antibody: Anti-Human IgG (Fab SPECIFIC) PEROXIDASE CONJUGATE Antibody developed in Goat Affinity Isolated Antibody (manufactured by SIGMA) solution 20000-fold diluted with TBST, at room temperature for 1 hr. The membrane was washed with TBST (15 min×1, 5 minx2), and made to emit light with ECL Western Blotting Detection Reagents (manufactured by GE Bioscience). The membrane that emitted light was exposed to light with an imager (ChemiDoc XRS; manufactured by Bio-Rad) for a suitable time.

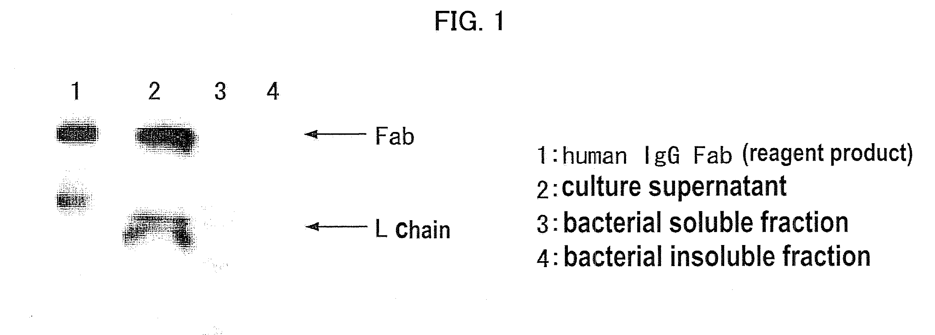

As a result, a band was observed near the assumed molecular weight (about 50 kDa) of Fab-type antibody, whereby it was confirmed that most of the Fab antibody was expressed by secretion in the culture supernatant (Fig. 1)

### (Example 9) Purification of Fab antibody from culture supernatant

Fab antibody was purified from a culture supernatant by the method shown below. The culture supernatant obtained in Example 7 was clarified with 0.2 µm filter (DISMIC; manufactured by TOYO filter paper), and applied to a protein G column (HiTrap ProteinG Fast Flow; manufactured by GE healthcare bioscience) equilibrated in advance with equilibration buffer (20 mM sodium phosphate, pH 7.0), and a purified Fab fraction was obtained from the fraction eluted with elution buffer (0.1M Glycine-HCl, pH 3.0). Furthermore, a concentrated solution was obtained by ultrafiltration using microcon 3 (manufactured by Millipore).

### (Example 10) Confirmation of Fab antibody

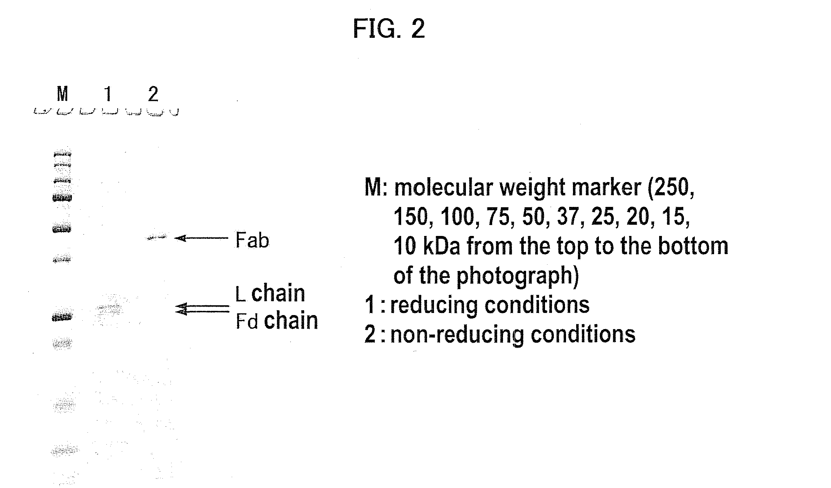

The Fab antibody obtained in Example 9 was analyzed by SDS-PAGE. That is, a mixture of 10 µl of the concentrated solution and an equal amount of 2×sample buffer was subjected to SDS-PAGE electrophoresis using 15% e-PAGEL (manufactured by ATTO) under non-reducing and reducing conditions (DTT in 2×sample buffer). After migration, the gel was washed with water for 15 min, stained with a staining solution (Bio-Safe CBB G-250 stain; manufactured by Bio-Rad) for 30 min, and decolorized with water. As a result, one band was confirmed near the molecular weight (about 50 kDa) of Fab antibody under non-reducing conditions, and two bands were confirmed near the molecular weight (about 25 kDa) of L chain and Fd chain under reducing conditions (Fig. 2).

### (Example 11) Confirmation of antigen binding ability of Fab-type antibody by ELISA method

The binding ability of Fab antibody expressed by secretion by Hansenula to antigen (TNF-α) was evaluated by the ELISA method. That is, TNF-α (manufactured by PEPROTECH) diluted with PBS was added to a plate for ELISA (MaxiSoap; manufactured by NUNC) at 50 µl/well, and incubated overnight at 4°C. After incubation, the solution in the well was removed, 5-fold diluted Immunoblock (manufactured by Dainippon Sumitomo Pharmaceuticals Co., Ltd.) was added at 250 µl/well, and the mixture was blocked by being stood at room temperature for 1 hr. The solution in the well was removed, and the cells were washed 3 times with PBST (PBS+0.1% Tween20). The concentrated solution obtained Example 9 was diluted and added at 50 µl/well, and the mixture was reacted at room temperature for 2 hr. After washing twice with PBST, a secondary antibody solution (secondary antibody: Anti-Human IgG (Fab SPECIFIC) PEROXIDASE CONJUGATE Antibody developed in Goat Affinity Isolated Antibody; manufactured by SIGMA) was 7500-fold diluted with PBSTIB (PBST+1/50 diluted immunoblock) solution and added by 50 µl per well, and the mixture was reacted at room temperature for 1 hr. After washing 4 times with PBST, 100 µl of TMB 1-Component Microwell Peroxidase Substrate, SureBlue (manufactured by KPL) was added, and the mixture was stood at room temperature for 20 min. 100 µl of TMB Stop Solution (manufactured by KPL) was added to discontinue the reaction, and the absorbance at 450 nm was measured by a microplatereader (BenchMark Plus; manufactured by Bio-Rad). As a result, increase of absorbance in proportion with the amount of the concentrated solution added was observed, whereby it was confirmed that the Fab antibody expressed by secretion by Hansenula had an antigen binding ability.

### (Example 12) Purification of full length antibody

A full length antibody was purified by the method shown below. The culture supernatant of the full length antibody expressing strain prepared in Example 7 was clarified with 0.2 µm filter (DISMIC; manufactured by TOYO filter paper), and applied to a protein A column (HiTrap rProtein A Fast Flow; manufactured by GE healthcare bioscience) equilibrated in advance with equilibration buffer (20 mM sodium phosphate, pH 7.0), and eluted with an elution buffer (0.1 M sodium citrate, pH 3.0) to purify a full length antibody.

### (Example 13) Detection of complete humanized anti TNF-α full length antibody by Western blotting

A complete humanized anti-TNF-α full length antibody was detected by the method shown below. That is, the full length antibody solution obtained in Example 12 and standard IgG (ImmunoPure Human IgG, whole Molecule (manufactured by Thermo scientific)) were detected by the Western blotting method using Anti-Human IgG-Fc HRP conjugate Antibody (manufactured by Bethyl Laboratories, Inc.) and Anti-Human IgG (Fab SPECIFIC) HRP conjugate Antibody (manufactured by SIGMA). First, a mixture of 10 µl of a sample and an equal amount of 2×sample buffer (10% SDS, 50% Glycerol, 0.3125M Tris-HCl, pH 6.8, 0.01% BPB) was subjected to SDS-PAGE electrophoresis using 15% e-PAGEL (manufactured by ATTO) under non-reducing and reducing conditions (condition with addition of 50 mM DTT to the aforementioned 2×sample buffer). Then, two sheets of filter paper immersed in anode buffer 1 (0.3M Tris, 10% Methanol, pH 10.4), one sheet of filter paper and one sheet of PVDF membrane (Immobilon-P; manufactured by Millipore) immersed in anode buffer 2 (25 mM Tris, 10% Methanol, pH 10.4), and the gel after electrophoresis and three sheets of filter paper immersed in cathode buffer (25 mM Tris, 192 mM glycine, 10% Methanol, pH 9.4) for 15 min were placed in this order on a semi-dry blotting apparatus (MilliBlot-Graphite Electroblotter I; manufactured by Millipore), and constant electric current of 120 mA was flown for 90 min to transfer the protein on the gel to the PVDF membrane. The membrane after transfer was blocked by immersing in TBST (0.24% Tris, 0.8% NaCl, pH 7.6, 0.1% Tween20) containing 3% BSA for 1 hr. The membrane was washed with TBST 15 min×1, 5 min×2 and reacted with a secondary antibody 50000-fold diluted with TBST: Anti-Human IgG-Fc HRP conjugate Antibody (manufactured by Bethyl Laboratories, Inc.) solution or a secondary antibody 20000-fold diluted with TBST: Anti-Human IgG (Fab SPECIFIC) HRP conjugate Antibody (manufactured by SIGMA) solution, at room temperature for 1 hr. The membrane was washed with TBST (15 min×1, 5 min×2), and made to emit light with ECL Western Blotting Detection Reagents (manufactured by GE Bioscience). The membrane that emitted light was exposed to light with an imager (ChemiDoc XRS; manufactured by Bio-Rad) for a suitable time. As a result, a band was observed at the same position as standard IgG under non-reducing conditions. Under reducing conditions, moreover, a band of H chain (about 50 kDa) was observed when Anti-Human IgG-Fc HRP conjugate Antibody was used as the secondary antibody, and a band of L chain (about 25 kDa) was observed when Anti-Human IgG (Fab SPECIFIC) HRP conjugate Antibody was used as the secondary antibody (Fig. 3).

### (Example 14) Analysis of copy number of introduced antibody expression vector on chromosome by Southern blotting

Southern blotting of genomic DNA was performed using DIG-High Prime DNA Labeling and Detection Starter Kit (manufactured by Roche). For detection of the respective Fab-type antibody expression vectors and L chain expression vectors described in Examples 1 - 4, LEU2 gene was used as a probe. For detection of Fd chain expression vector described in Example 3, URA3 gene was used as a probe. The genomic DNA of each antibody expression strain prepared in Example 5 was digested with restriction enzyme (BglII), and applied to agarose gel electrophoresis. The gel was immersed in a hydrolysis solution (0.25M HCl) for 30 min, and washed with water. Then, it was immersed in a denaturation solution (1.5M NaCl, 0.5M NaOH) for 30 min, and washed with water. Then, it was immersed in a neutralization solution (1.5M NaCl, 0.5M Tris-HCl, pH 7.2, 1 mM Na2 EDTA) for 30 min. The gel was neutralized and the genomic DNA was transferred from the neutralized gel on a nylon membrane (Nylon membrane, positively charged; manufactured by Roche) overnight by using 10×SSC (1.5M NaCl, 0.15M sodium citrate) as a developing solution. The membrane was washed with 6×SSC for 20 min at room temperature, dried and subjected to a baking treatment at 80°C for 2 hr. The membrane treatments thereafter (probe preparation, prehybridization, hybridization, probe detection) were performed under the conditions described in the aforementioned kit. The membrane after luminescence of the probe was exposed to light by an imager (ChemiDoc XRS; manufactured by Bio-Rad) for a suitable time. The number of introduced copies of each F expression vector was calculated by calculating the relative value of the number and intensity of the bands of the vector-derived LEU2 gene or URA3 gene relative to the band intensity of LEU2 gene or URA3 gene on Hansenula polymorpha chromosome (one copy is present on the chromosome). As a result, the number of the introduced copies of Fab-type antibody expression vector and L chain expression vector using LEU2 gene and URA3 gene as a selection marker was 1. The number of the introduced copies of L chain expression vector and Fd chain expression vector using LEU2 gene and URA3 gene with shortened promoter region as a selection marker was 2 - 3.

### Industrial Applicability

Using the transformant of Hansenula polymorpha of the present invention, a desired antibody pharmaceutical product can be produced conveniently and at a low cost as compared to the conventional antibody production methods including cultivation of animal cells.
This application is based on patent application No. 2009-239471 filed in Japan (filing date: October 16, 2009), the contents of which are incorporated in full herein.

## Claims

1. A transformant of Hansenula polymorpha that produces an antibody and/or a partial antibody.

2. The transformant according to claim 1, comprising an expression vector comprising an antibody gene and/or a partial antibody gene incorporated in a linear state into a chromosome.

3. The transformant according to claim 2, wherein the expression vector has a promoter and a base sequence encoding a signal sequence on the 5'-side, and a termination codon and/or a terminator sequence on the 3'-side, of the antibody gene and/or the partial antibody gene.

4. The transformant according to claim 2 or 3, wherein the expression vector comprises a homology base sequence with not less than 50% of sequence identity with not less than 50 bp base sequence contained in the chromosome of Hansenula polymorpha.

5. The transformant according to claim 4, wherein the homology base sequence is the promoter sequence present on the 5'-side of the antibody gene and/or the partial antibody gene, and/or the terminator sequence present on the 3'-side of the antibody gene and/or the partial antibody gene.

6. The transformant according to any one of claims 2 to 5, wherein the number of copies of the expression vector per chromosome is not less than 2 copies.

7. The transformant according to any one of claims 2 to 6, wherein the expression vector is incorporated into the chromosome in an MOX terminator region and/or an MOX promoter region on the chromosome.

8. The transformant according to any one of claims 2 to 7, wherein the function of the MOX gene on the chromosome is partially or completely deficient.

9. The transformant according to any one of claims 1 to 8, which is selected by using an auxotrophic complementary gene and/or a drug resistance gene as a selection marker.

10. The transformant according to claim 9, wherein the auxotrophic complementary gene and/or the drug resistance gene are/is one or more genes selected from the group consisting of URA3, LEU2, ADE1, HIS4, G418 resistance gene, zeocin resistance gene and hygromycin resistance gene.

11. The transformant according to any one of claims 2 to 10, wherein the partial antibody gene contained in the expression vector is a Fab-type antibody gene.

12. The transformant according to claim 11, wherein the expression vector containing the Fab-type antibody gene is a base sequence containing an L chain gene and an Fd chain gene, a base sequence containing an L chain Fd chain fusion gene and/or a base sequence containing an Fd chain L chain fusion gene.

13. The transformant according to any one of claims 1 to 12, which secretes an antibody and/or a partial antibody.

14. The transformant according to any one of claims 1 to 13, wherein the antibody and/or the partial antibody are/is an antibody selected from a human antibody, a humanized antibody, a chimera antibody and a mouse antibody and/or a partial antibody derived therefrom.

15. The transformant according to any one of claims 1 to 14, wherein the antibody and/or the partial antibody are/is bound to one or more antigens selected from the group consisting of CD20, HER2, IL2R, CD33, CD52, EGFR, VEGF, CD3, CD25, TNFα, CD11, IgE, CD2, α4integrin, CD80, CD86, IL6R, C5a, GPIIb/IIIa, RSVF Protein, VEGF-A and GM-CSF.

16. The transformant according to any one of claims 13 to 15, wherein the partial antibody is a Fab-type antibody.

17. The transformant according to any one of claims 13 to 16, wherein the secretory production amount of the Fab-type partial antibody in a culture supernatant is not less than 1 mg/mL when a Fab-type partial antibody derived from the antibody contained in HUMIRA (registered trade mark) is produced as the antibody and/or the partial antibody.

18. A production method of an antibody and/or a partial antibody, comprising using the transformant according to any one of claims 1 to 17.

19. An antibody and/or a partial antibody produced by the production method according to claim 18.


SEQUENCE LISTING

<110>  KANEKA Corporation

<120>  Hansenula polymorpha capable of producing antibody, method of
producing antibody by using the same, and antibody produced by the method

<130>  091620

<150>  JP 2009-239471
<151>  2009-10-16

<160>  36

<170>  PatentIn version 3.1

<210>  1
<211>  1510
<212>  DNA
<213>  Hansenula polymorpha

<400>  1
tcgacgcgga gaacgatctc ctcgagctgc tcgcggatca gcttgtggcc cggtaatgga      60

accaggccga cggcacgctc cttgcggacc acggtggctg gcgagcccag tttgtgaacg     120

aggtcgttta gaacgtcctg cgcaaagtcc agtgtcagat gaatgtcctc ctcggaccaa     180

ttcagcatgt tctcgagcag ccatctgtct ttggagtaga agcgtaatct ctgctcctcg     240

ttactgtacc ggaagaggta gtttgcctcg ccgcccataa tgaacaggtt ctctttctgg     300

tggcctgtga gcagcgggga cgtctggacg gcgtcgatga ggcccttgag gcgctcgtag     360

tacttgttcg cgtcgctgta gccggccgcg gtgacgatac ccacatagag gtccttggcc     420

attagtttga tgaggtgggg caggatgggc gactcggcat cgaaattttt gccgtcgtcg     480

tacagtgtga tgtcaccatc gaatgtaatg agctgcagct tgcgatctcg gatggttttg     540

gaatggaaga accgcgacat ctccaacagc tgggccgtgt tgagaatgag ccggacgtcg     600

ttgaacgagg gggccacaag ccggcgtttg ctgatggcgc ggcgctcgtc ctcgatgtag     660

aaggcctttt ccagaggcag tctcgtgaag aagctgccaa cgctcggaac cagctgcacg     720

agccgagaca attcgggggt gccggctttg gtcatttcaa tgttgtcgtc gatgaggagt     780

tcgaggtcgt ggaagatttc cgcgtagcgg cgttttgcct cagagtttac catgaggtcg     840

tccactgcag agatgccgtt gctcttcacc gcgtacagga cgaacggcgt ggccagcagg     900

cccttgatcc attctatgag gccatctcga cggtgttcct tgagtgcgta ctccactctg     960

tagcgactgg acatctcgag actgggcttg ctgtgctgga tgcaccaatt aattgttgcc    1020

gcatgcatcc ttgcaccgca agtttttaaa acccactcgc tttagccgtc gcgtaaaact    1080

tgtgaatctg gcaactgagg gggttctgca gccgcaaccg aacttttcgc ttcgaggacg    1140

cagctggatg gtgtcatgtg aggctctgtt tgctggcgta gcctacaacg tgaccttgcc    1200

taaccggacg gcgctaccca ctgctgtctg tgcctgctac cagaaaatca ccagagcagc    1260

agagggccga tgtggcaact ggtggggtgt cggacaggct gtttctccac agtgcaaatg    1320

cgggtgaacc ggccagaaag taaattctta tgctaccgtg cagcgactcc gacatcccca 1380

gtttttgccc tacttgatca cagatggggt cagcgctgcc gctaagtgta cccaaccgtc 1440

cccacacggt ccatctataa atactgctgc cagtgcacgg tggtgacatc aatctaaagt 1500

acaaaaacaa 1510

<210> 2
<211> 606
<212> DNA
<213> Hansenula polymorpha

<400> 2
gagacgtgga aggacatacc gcttttgaga agcgtgtttg aaaatagttc tttttctggt 60

ttatatcgtt tatgaagtga tgagatgaaa agctgaaata gcgagtatag gaaaatttaa 120

tgaaaattaa attaaatatt ttcttaggct attagtcacc ttcaaaatgc cggccgcttc 180

taagaacgtt gtcatgatcg acaactacga ctcgtttacc tggaacctgt acgagtacct 240

gtgtcaggag ggagccaatg tcgaggtttt caggaacgat cagatcacca ttccggagat 300

tgagcagctc aagccggacg ttgtggtgat atcccctggt cctggccatc caagaacaga 360

ctcgggaata tctcgcgacg tgatcagcca ttttaaaggc aagattcctg tctttggtgt 420

ctgtatgggc cagcagtgta tcttcgagga gtttggcgga gacgtcgagt atgcgggcga 480

gattgtccat ggaaaaacgt ccactgttaa gcacgacaac aagggaatgt tcaaaaacgt 540

tccgcaagat gttgctgtca ccagatacca ctcgctggcc ggaacgctca agtcgcttcc 600

ggactg 606

<210> 3
<211> 1340
<212> DNA
<213> Hansenula polymorpha

<400> 3
gagtccctga gtacgtaagc ggtttggtaa tacgaaataa aaagacagga atgagtaagt 60

ggatggtttt tcaatttttc cggtaccggc gcaaaatagt tgcatcattt tgcaatcatg 120

agtaagaaca ttgtgcttct ccctggtgat cacgtgggcc ccgaggttgt tgcggaggcc 180

gtcaaggttc tcgaggctgt ctcgtcggca atcggcgtta agttcaactt ttccaagcac 240

ctgatcggcg gtgcctcgat cgatgcttat ggggtgccat tgtccgacga ggccctcgaa 300

gccgccaaga aggctgacgc cgttttgctt ggagccgtcg gaggacctaa gtggggaacc 360

ggctccgtgc gtcctgagca gggtctgttg aagatcagaa aagagctcaa cttgtacgcg 420

aacctgcgtc catgcagttt tgcttccgac gctcttctga agctatctcc actgaaatca 480

gaaatcgtca agggcactga ctttgttgtt gtgcgtgagt tggttggtgg aatctacttt 540

ggtgaccgca aggaggatgc cggcgacgga gttgccagcg acactgagag ctattctgtt 600

ccagaggtgc agagaatcac gagaatggcg gcctttttgg cgctgcagag cgacccaccg 660

ctcccactgt ggtcgctgga caaagccaac gtgcttgcat cctcgcgttt gtggcggaag 720

actgttgagg agaccatcaa gaacgagttc ccgcagctga cggtgcagca ccagctgatc 780

gactcggcag ccatgatttt ggtcaagtcg ccaacgaaac tcaacggtgt cattgtcacc 840

aacaacatgt ttggcgacat catcagtgac gaggccagtg tgattcctgg gtctctgggc 900

ctgctgcctt ctgcctcgtt ggcgtctctg ccagacacaa acaaggcgtt tggtctttac 960

gagccctgcc acggctcggc gccagatttg ggcccgggca aggtcaatcc attggccaca 1020

attttgtctg ccgccatgat gctgaagctg tcgctggact tggtggatgc cggccgtgcg 1080

atcgagcagg ccgtcaagaa cgtcctggat gcaggtatca tgactgccga tttgggtgga 1140

agctcctcaa cacaggaagt tggtgatgct gttgcgcagg aggtggccaa gctactcaag 1200

aactaaataa gggagaaaaa aaagtaggat ctcgaataat tcctaaataa tcccaaaaat 1260

cctaaacacg cacgcctcac agattttatt tttttcgacg cgacgctcta ttgtttattt 1320

tttagctttt ccatgtcaac 1340

<210> 4
<211> 1295
<212> DNA
<213> Hansenula polymorpha

<400> 4
caggaatgag taagtggatg gtttttcaat ttttccggta ccggcgcaaa atagttgcat 60

cattttgcaa tcatgagtaa gaacattgtg cttctccctg gtgatcacgt gggccccgag 120

gttgttgcgg aggccgtcaa ggttctcgag gctgtctcgt cggcaatcgg cgttaagttc 180

aacttttcca agcacctgat cggcggtgcc tcgatcgatg cttatggggt gccattgtcc 240

gacgaggccc tcgaagccgc caagaaggct gacgccgttt tgcttggagc cgtcggagga 300

cctaagtggg gaaccggctc cgtgcgtcct gagcagggtc tgttgaagat cagaaaagag 360

ctcaacttgt acgcgaacct gcgtccatgc agttttgctt ccgacgctct tctgaagcta 420

tctccactga aatcagaaat cgtcaagggc actgactttg ttgttgtgcg tgagttggtt 480

ggtggaatct actttggtga ccgcaaggag gatgccggcg acggagttgc cagcgacact 540

gagagctatt ctgttccaga ggtgcagaga atcacgagaa tggcggcctt tttggcgctg 600

cagagcgacc caccgctccc actgtggtcg ctggacaaag ccaacgtgct tgcatcctcg 660

cgtttgtggc ggaagactgt tgaggagacc atcaagaacg agttcccgca gctgacggtg 720

cagcaccagc tgatcgactc ggcagccatg attttggtca agtcgccaac gaaactcaac 780

ggtgtcattg tcaccaacaa catgtttggc gacatcatca gtgacgaggc cagtgtgatt 840

cctgggtctc tgggcctgct gccttctgcc tcgttggcgt ctctgccaga cacaaacaag 900

gcgtttggtc tttacgagcc ctgccacggc tcggcgccag atttgggccc gggcaaggtc 960

aatccattgg ccacaatttt gtctgccgcc atgatgctga agctgtcgct ggacttggtg 1020

gatgccggcc gtgcgatcga gcaggccgtc aagaacgtcc tggatgcagg tatcatgact 1080

gccgatttgg gtggaagctc ctcaacacag gaagttggtg atgctgttgc gcaggaggtg 1140

gccaagctac tcaagaacta aataagggag aaaaaaaagt aggatctcga ataattccta 1200

aataatccca aaaatcctaa acacgcacgc ctcacagatt ttattttttt cgacgcgacg 1260

ctctattgtt tattttttag cttttccatg tcaac 1295

<210> 5
<211> 993
<212> DNA
<213> Hansenula polymorpha

<400> 5
ttgctgttct ttatagttga gttgatccat ggtgtatcaa aaataaacta atgaattatc 60

gaaaaatttt caacatttcc ctgaataata atcaacatgt ataaatctta tggagaaagg 120

gcgaagtctc acccatctaa ggtcgccagc agactactta atttgatgga atccaagcaa 180

acaaacctct gcgcttctgt ggatgtgact aaaactcagg aattattgga gcttcttgat 240

aaactgggcc cttacatctg ccttgtcaaa actcatattg acatagtaga ggacttctct 300

tatgaacaca ccattttacc attacaagga cttgcaaaga aacacaactt catgattttt 360

gaagacagaa agtttgctga tataggaaac acagtcaaac tacagtataa gggaggaatt 420

tatcgaacat ccaagtgggc cgatatcacg aatgcacacg gagtgactgg tgcaggaatt 480

gttgaaggtc ttaaacaggc cgcagaagaa agtacagatg agccacgtgg gcttttgatg 540

cttgctgagc tctcttcaaa gggatcatta gctaccggtg agtatactca aaaaactgtg 600

gaaatagcga aaagcgataa agaatttgtc attggattta ttgcacagag agacatggga 660

ggtcgtgagg aaggctttga ctggctgatc atgactccag gagttggttt agatgataaa 720

ggcgattctc tgggccaaca gtacagaact gttgatgaag tgatgcaaac aggaaccgat 780

gtcattatcg ttggaagagg tttattcgga aaaggaagag atcctgaagt ggaagggaag 840

agatacagaa atgctgggtg ggaagcttac aagcggcgca ttgcttaacg gctttcagtt 900

ctatatacat cgtcaaaatt gattttcgct aaaatgctga cgggatattt cgaatacgaa 960

aagcccaata gaagtcgcgg gaatactagt gaa 993

<210> 6
<211> 933
<212> DNA
<213> Hansenula polymorpha

<400> 6
gaaaaatttt caacatttcc ctgaataata atcaacatgt ataaatctta tggagaaagg 60

gcgaagtctc acccatctaa ggtcgccagc agactactta atttgatgga atccaagcaa 120

acaaacctct gcgcttctgt ggatgtgact aaaactcagg aattattgga gcttcttgat 180

aaactgggcc cttacatctg ccttgtcaaa actcatattg acatagtaga ggacttctct 240

tatgaacaca ccattttacc attacaagga cttgcaaaga aacacaactt catgattttt 300

gaagacagaa agtttgctga tataggaaac acagtcaaac tacagtataa gggaggaatt 360

tatcgaacat ccaagtgggc cgatatcacg aatgcacacg gagtgactgg tgcaggaatt 420

gttgaaggtc ttaaacaggc cgcagaagaa agtacagatg agccacgtgg gcttttgatg 480

cttgctgagc tctcttcaaa gggatcatta gctaccggtg agtatactca aaaaactgtg 540

gaaatagcga aaagcgataa agaatttgtc attggattta ttgcacagag agacatggga 600

ggtcgtgagg aaggctttga ctggctgatc atgactccag gagttggttt agatgataaa 660

ggcgattctc tgggccaaca gtacagaact gttgatgaag tgatgcaaac aggaaccgat 720

gtcattatcg ttggaagagg tttattcgga aaaggaagag atcctgaagt ggaagggaag 780

agatacagaa atgctgggtg ggaagcttac aagcggcgca ttgcttaacg gctttcagtt 840

ctatatacat cgtcaaaatt gattttcgct aaaatgctga cgggatattt cgaatacgaa 900

aagcccaata gaagtcgcgg gaatactagt gaa 933

<210> 7
<211> 290
<212> DNA
<213> Hansenula polymorpha

<400> 7
gctatacaga gctttatatc accttactga acgctagagt agacccaatt cccggctcac 60

accaccctta catgcagagc taaccaataa ggtaattaat taacactata tagctcgtgg 120

tgaacactgg cccggagtag tcatacgtgt aggtttttgg cgtgatgaaa atcaggtggc 180

gcacgacttt tcgtaaagtt cgggagggag tgctgcaaac ggcatataag gaccagtttt 240

tctcgcacat tatcaattgc tctttagtac aaagataata tagaaaccat 290

<210> 8
<211> 255
<212> DNA
<213> Saccharomyces cerevisiae

<400> 8
atgagatttc cttcaatttt tactgcagtt ttattcgcag catcctccgc attagctgct 60

ccagtcaaca ctacaacaga agatgaaacg gcacaaattc cggctgaagc tgtcatcggt 120

tactcagatt tagaagggga tttcgatgtt gctgttttgc cattttccaa cagcacaaat 180

aacgggttat tgtttataaa tactactatt gccagcattg ctgctaaaga agaaggggta 240

tctctcgaga aaaga 255

<210> 9
<211> 645
<212> DNA

<213>  Homo sapiens

<400>  9
gacatccaga tgacccagtc tccatcctcc ctgtctgcat ctgtagggga cagagtcacc      60

atcacttgtc gggcaagtca gggcatcaga aattacttag cctggtatca gcaaaaacca     120

gggaaagccc ctaagctcct gatctatgct gcatccactt tgcaatcagg ggtcccatct     180

cggttcagtg gcagtggatc tgggacagat ttcactctca ccatcagcag cctacagcct     240

gaagatgttg caacttatta ctgtcaaagg tataaccgtg caccgtatac ttttggccag     300

gggaccaagg tggaaatcaa acgaactgtg gcggcgccat ctgtcttcat cttcccgcca     360

tctgatgagc agttgaaatc tggaactgcc tctgttgtgt gcctgctgaa taacttctat     420

cctcgagagg ccaaagtaca gtggaaggtg gataacgccc tccaatcggg taactcccag     480

gagagtgtca cagagcagga cagcaaggac agcacctaca gcctcagcag caccctgacg     540

ctgagcaaag cagattacga gaaacacaaa gtctacgcct gcgaagtcac ccatcagggc     600

ctgagctcgc ccgtcacaaa gagcttcaac aggggagagt gttag                     645


<210>  10
<211>  1356
<212>  DNA
<213>  Homo sapiens

<400>  10
gaggtgcagc tggtggagtc tgggggaggc ttggtacagc ccggcaggtc cctgagactc      60

tcctgtgcgg cctctggatt cacctttgat gattatgcca tgcactgggt ccggcaagct     120

ccagggaagg gcctggaatg ggtctcagct atcacttgga atagtggtca catagactat     180

gcggactctg tggagggccg attcaccatc tccagagaca acgccaagaa ctccctgtat     240

ctgcaaatga acagtctgag agctgaggat acggccgtat attactgtgc gaaagtctcg     300

taccttagca ccgcgtcctc ccttgactat tggggccaag gaaccctggt caccgtctcg     360

agtgctagct tcaagggccc atcggtcttc cccctggcac cctcctccaa gagcacctct     420

gggggcacag cggccctggg ctgcctggtc aaggactact tccccgaacc ggtgacggtg     480

tcgtggaact caggcgccct gaccagcggc gtgcacacct tcccggctgt cctacagtcc     540

tcaggactct actccctcag cagcgtggtg accgtgccct ccagcagctt gggcacccag     600

acctacatct gcaacgtgaa tcacaagccc agcaacacca aggtggacaa gagagttgag     660

cccaaatctt gtgacaaaac tcacacatgc ccaccgtgcc cagcacctga actcctgggg     720

ggaccgtcag tcttcctctt ccccccaaaa cccaaggaca ccctcatgat ctcccggacc     780

cctgaggtca catgcgtggt ggtggacgtg agccacgaag accctgaggt caagttcaac     840

tggtacgtgg acggcgtgga ggtgcataat gccaagacaa agccgcggga ggagcagtac     900

aacagcacgt accgtgtggt cagcgtcctc accgtcctgc accaggactg gctgaatggc     960

aaggagtaca agtgcaaggt ctccaacaaa gccctcccag cccccatcga gaaaaccatc    1020

tccaaagcca aagggcagcc ccgagaacca caggtgtaca ccctgccccc atcccgggag 1080

gagatgacca agaaccaggt cagcctgacc tgcctggtca aaggcttcta tcccagcgac 1140

atcgccgtgg agtgggagag caatgggcag ccggagaaca actacaagac cacgcctccc 1200

gtgctggact ccgacggctc cttcttcctc tatagcaagc tcaccgtgga caagagcagg 1260

tggcagcagg ggaacgtctt ctcatgctcc gtgatgcatg aggctctgca caaccactac 1320

acgcagaaga gcctctccct gtctccgggt aaatga 1356

<210> 11
<211> 50
<212> DNA
<213> Artificial

<220>
<223> artificial

<400> 11
aagcttgcgg ccgcggatcc actagtcaat tgagatcttc tagagaattc 50

<210> 12
<211> 30
<212> DNA
<213> Artificial

<220>
<223> artificial

<400> 12
ataaaagctt gagtccctga gtacgtaagc 30

<210> 13
<211> 30
<212> DNA
<213> Artificial

<220>
<223> artificial

<400> 13
tataaagctt gttgacatgg aaaagctaaa 30

<210> 14
<211> 30
<212> DNA
<213> Artificial

<220>
<223> artificial

<400> 14
ataaggatcc tcgacgcgga gaacgatctc 30

<210> 15
<211> 30
<212> DNA

<213>  Artificial

<220>
<223>  artificial

<400>  15
tataggatcc ttgtttttgt actttagatt                                        30


<210>  16
<211>  30
<212>  DNA
<213>  Artificial

<220>
<223>  artificial

<400>  16
ataacaattg tcgacgcgga gaacgatctc                                        30


<210>  17
<211>  30
<212>  DNA
<213>  Artificial

<220>
<223>  artificial

<400>  17
tatacaattg ttgtttttgt actttagatt                                        30


<210>  18
<211>  30
<212>  DNA
<213>  Artificial

<220>
<223>  artificial

<400>  18
ataatctaga gagacgtgga aggacatacc                                        30


<210>  19
<211>  30
<212>  DNA
<213>  Artificial

<220>
<223>  artificial

<400>  19
tatatctaga cagtccggaa gcgacttgag                                        30


<210>  20
<211>  30
<212>  DNA
<213>  Artificial

<220>
<223>  artificial

<400>  20
ataaactagt atgagatttc cttcaatttt                                        30


<210>  21
<211>  23
<212>  DNA
<213>  Artificial

<220>
<223>  artificial

<400>  21
tcttttctcg agagataccc ctt                                               23


<210>  22
<211>  40
<212>  DNA
<213>  Artificial

<220>
<223>  artificial

<400>  22
gggtatctct cgagaaaaga gacatccaga tgacccagtc                             40


<210>  23
<211>  30
<212>  DNA
<213>  Artificial

<220>
<223>  artificial

<400>  23
tataactagt ctaacactct cccctgttga                                        30


<210>  24
<211>  30
<212>  DNA
<213>  Artificial

<220>
<223>  artificial

<400>  24
ataaagatct atgagatttc cttcaatttt                                        30


<210>  25
<211>  40
<212>  DNA
<213>  Artificial

<220>
<223>  artificial

<400>  25
gggtatctct cgagaaaaga gaggtgcagc tggtggagtc                             40


<210>  26

<211>  30
<212>  DNA
<213>  Artificial

<220>
<223>  artificial

<400>  26
tataagatct ttaacaagat ttgggctcaa                                        30


<210>  27
<211>  30
<212>  DNA
<213>  Artificial

<220>
<223>  artificial

<400>  27
ataaggatcc gctatacaga gctttatatc                                        30


<210>  28
<211>  30
<212>  DNA
<213>  Artificial

<220>
<223>  artificial

<400>  28
tataggatcc atggtttcta tattatcttt                                        30


<210>  29
<211>  30
<212>  DNA
<213>  Artificial

<220>
<223>  artificial

<400>  29
ataacaattg gctatacaga gctttatatc                                        30


<210>  30
<211>  30
<212>  DNA
<213>  Artificial

<220>
<223>  artificial

<400>  30
tatacaattg atggtttcta tattatcttt                                        30


<210>  31
<211>  30
<212>  DNA
<213>  Artificial

<220>

<223>  artificial

<400>  31
ataaaagctt caggaatgag taagtggatg                                        30


<210>  32
<211>  30
<212>  DNA
<213>  Artificial

<220>
<223>  artificial

<400>  32
tataagatct ctaacactct cccctgttga                                        30


<210>  33
<211>  30
<212>  DNA
<213>  Artificial

<220>
<223>  artificial

<400>  33
ataagaattc ttgctgttct ttatagttga                                        30


<210>  34
<211>  30
<212>  DNA
<213>  Artificial

<220>
<223>  artificial

<400>  34
tatagaattc ttcactagta ttcccgcgac                                        30


<210>  35
<211>  30
<212>  DNA
<213>  Artificial

<220>
<223>  artificial

<400>  35
ataagaattc gaaaaatttt caacatttcc                                        30


<210>  36
<211>  30
<212>  DNA
<213>  Artificial

<220>
<223>  artificial

<400>  36
tataagatct tcatttaccc ggagacaggg                                        30